# EUROPEAN PATENT APPLICATION

(11) **EP 4 091 658 A1**
(43) Date of publication of application: **23.11.2022**
(21) Application number: 20914635.6
(22) Date of filing: 16.01.2020
(51) Int. Cl.: A61M 25/08, A61M 25/09

(54) **MEDICAL TOOL GRIPPER**

(71) Applicant: Asahi Intecc Co., Ltd., Seto-shi, Aichi 489-0071 (JP)
(72) Inventor: NANTO, Shinsuke, Nishinomiya-shi, Hyogo 662-0018 (JP); UJO, Ren, Seto-shi, Aichi 489-0071 (JP); OSHIMIZU, Ryuya, Seto-shi, Aichi 489-0071 (JP)
(74) Representative: Prinz & Partner mbB
(86) International application number: PCT/JP2020/001271
(87) International publication number: WO 2021/144915

(57) **Abstract**

To suppress the case in which a part of a medical tool comes out to the outside against the intension of a technician such as a doctor, in a medical tool gripper for gripping a long medical tool.

A medical tool gripper for gripping a long medical tool includes a substantially hollow cylindrical main body and a fixing tool for fixing a medical tool to the main body. The main body has main body inner space that extends over the entire longitudinal direction of the main body and in which the medical tool can be arranged, and a main body groove that is open on the outer periphery of the main body and is communicated with the main body inner space over the entire longitudinal length of the main body. The main body groove includes a distal end portion including the distal end of the main body groove, and a proximal end portion including the proximal end of the main body groove and having an opening at a different position from an opening of the distal end portion in the circumferential direction of the main body.

## Description

### TECHNICAL FIELD

The technology disclosed in the present specification relates to a medical tool gripper for gripping a long medical tool.

### BACKGROUND

In general, a guide wire is inserted into a body in the method of treating or examining a constricted part or an occluded part in a blood vessel or the like. The technician such as a doctor generally operates a guide wire by rotating a torque device attached on the proximal end side of the guide wire and thus rotating the guide wire (see Patent Literature 1, for example).

The torque device described in Patent Literature 1 includes a substantially hollow cylindrical main body, and a fixing tool for fixing a guide wire to the main body. The main body has substantially linear inner space that extends over the entire longitudinal length of the main body and in which a guide wire can be arranged, and a groove that is open on the outer periphery of the main body and is communicated with the inner space over the entire longitudinal length of the main body. The opening of the groove is formed linearly on the outer periphery of the main body, over the entire longitudinal length of the main body.

### CITATION LIST

### Patent Literature

Patent Literature 1: Japanese Patent No. 5030949

### SUMMARY

### Technical Problem

In the torque device described in Patent Literature 1, the opening of the above-described groove formed in the main body is formed linearly over the entire longitudinal length of the main body on the outer periphery of the main body, as described above. Therefore, in a case where a guide wire is fixed to the main body by the fixing tool, a part of the guide wire may come out to the outside of the main body through such a groove against the intention of a technician such as a doctor, which may impede the operation of the guide wire or the like by the technician. This problem occurs not only in the torque device for gripping a guide wire, but commonly occurs in any medical tool grippers for gripping a long medical tool.

### Solution to Problem

The technology disclosed herein aims at solving the above-described problem, and the problem can be solved by the following aspects, for example.
(1) A medical tool gripper for gripping a long medical tool, the medical tool gripper including a substantially hollow cylindrical main body that includes main body inner space in which the medical tool can be arranged, and a fixing tool that fixes the medical tool to the main body, in which the main body has a main body groove that extends over an entire longitudinal direction of the main body, is communicated with the main body inner space, and is open on an outer periphery of the main body, and the main body groove includes a distal end portion including a distal end of the main body groove and a proximal end portion including a proximal end of the main body groove and having an opening at a different position from an opening of the distal end portion in a circumferential direction of the main body. In the medical tool gripper, the position in the circumferential direction of the opening of the proximal end portion is different from that of the opening of the distal end portion in the main body groove. Thus, it is possible to suppress the problem that the rear end portion of a medical tool comes out to the outside of the medical tool gripper through the main body groove, as compared with the case in which the opening of the main body groove is formed linearly over the entire longitudinal length of the main body on the outer periphery of the main body. Therefore, it is possible to prevent the operation of the medical tool against the intention of a technician.
(2) The medical tool gripper according to the above-described (1), in which the opening of the distal end portion and the opening of the proximal end portion of the main body groove may be linear along the longitudinal direction, and the main body groove may further include a communication portion communicating the opening of the distal end portion and the opening of the proximal end portion. In the medical tool gripper, the medical tool is arranged in the main body inner space through the main body groove having such a configuration, whereby it is possible to more reliably secure the straightness of the medical tool arranged in the main body inner space.
(3) The medical tool gripper according to the above-described (2), in which an opening of the communication portion may be extended in a direction inclined relative to the longitudinal direction such that the closer to the proximal end of the main body, the closer a position of the opening in a direction orthogonal to the longitudinal direction is to the opening of the proximal end portion of the main body groove. In the medical tool gripper, when the medical tool is inserted into the main body groove, it is possible to suppress the deterioration of the straightness of the medical tool and more reliably secure the straightness of the medical tool arranged in the main body inner space.
(4) The medical tool gripper according to any one of the above-described (1) to (3), in which the main body may include a first component that includes a distal end of the main body and in which the distal end portion of the main body groove is formed, and a second component connected to the first component that includes a proximal end of the main body and in which the proximal end portion of the main body groove is formed. Depending on a production process, easier production may be possible as compared with the case in which the main body is formed integrally.
(5) The medical tool gripper according to any one of the above-described (1) to (3), in which the main body may be formed integrally by molding. In the medical tool gripper, it is possible to reduce the number of members as compared with the case in which the main body is formed by a plurality of separate members.
(6) According to the above-described medical tool gripper, further including a substantially hollow cylindrical extension member that is connected to a proximal end side of the main body, in which the extension member include extension inner space that extends over the entire longitudinal length and in which the medical tool can be arranged and an extension groove that is open on the outer periphery of the main body, and is communicated with the extension inner space over the entire longitudinal length of the main body, and a position of an opening of a proximal end portion of the extension groove may be different in the circumferential direction of the main body from a position of an opening of the proximal portion of the main body groove. In the medical tool gripper, with such an extension member, it is possible to increase the length of the medical tool gripper itself. Furthermore, the position of the opening of the proximal end portion of the medical tool gripper with the extension member attached thereto (the proximal end portion of the extension groove) is different from the position of the opening of the proximal end portion of the main body without the extension member attached thereto. Thus, it is possible to more securely suppress the problem that the rear end portion of a medical tool comes out to the outside of the medical tool gripper through the main body groove.

The technology disclosed herein can be achieved in various aspects, such as medical tool grippers and methods for producing the same.

### BRIEF DISCRIPTION OF DRAWINGS

FIG. 1 is a perspective view illustrating an external appearance configuration of a torque device without a guide wire fixed thereto according to a first embodiment;
FIG. 2 is a side view illustrating the external appearance configuration of FIG. 1;
FIG. 3 is a transverse sectional view of the torque device along III-III of FIG. 2;
FIG. 4 is a diagram for explaining the form of the opening of a groove along the longitudinal direction of the main body;
FIG. 5 is a transverse sectional view of the torque device along IV-IV of FIG. 2;
FIG. 6 is a transverse sectional view of the torque device along V-V of FIG. 2;
FIG. 7 is a perspective longitudinal sectional view of the torque device illustrated in FIG. 1;
FIG. 8 is a transverse sectional view of the torque device along VII-VII of FIG. 2;
FIG. 9 is a perspective transverse sectional view of a torque device (a transverse section around a small diameter portion of a second component);
FIG. 10 is a perspective view illustrating an external appearance configuration of the torque device with a guide wire fixed thereto according to the first embodiment;
FIG. 11 is a perspective view illustrating an external appearance configuration of a torque device according to a second embodiment; and
FIG. 12 is a perspective view illustrating an external appearance configuration of a torque device according to a third embodiment.

### DESCRIPTION OF EMBODIMENTS

### A. First embodiment

### A-1. Configuration of torque device

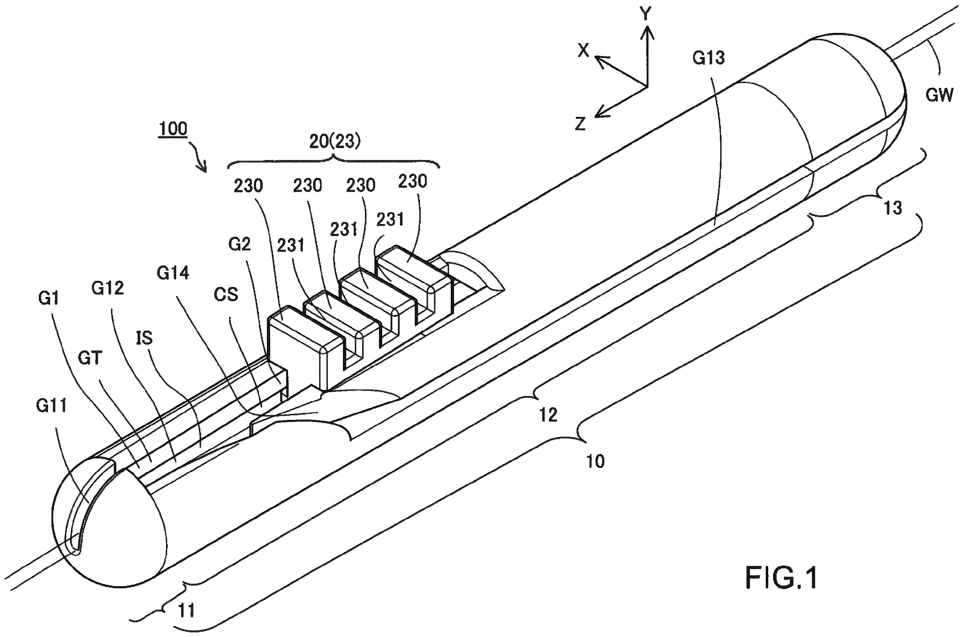

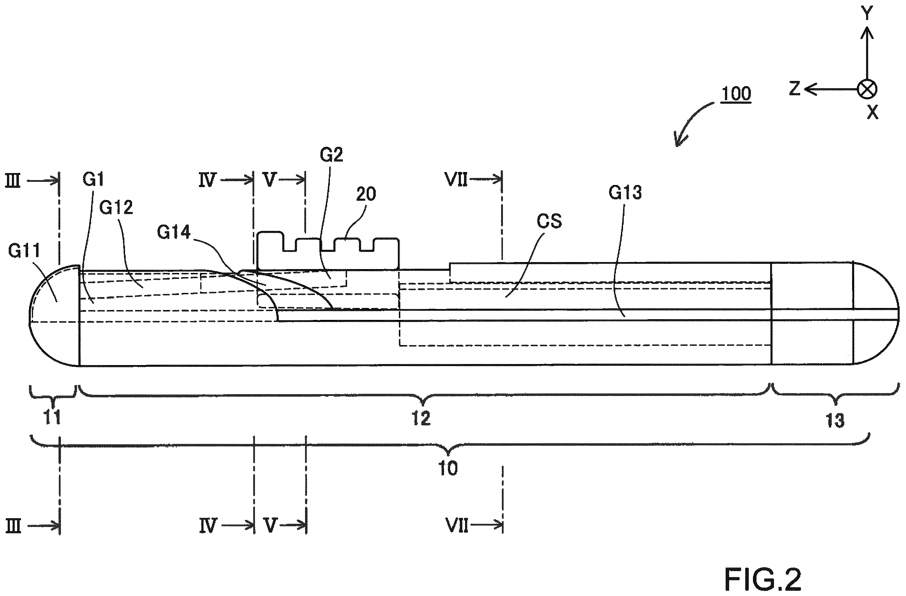

FIG. 1 is a perspective view illustrating an external appearance configuration of a torque device without a guide wire fixed thereto according to the first embodiment. FIG. 1 illustrates XYZ axes for specifying a direction, the XYZ being orthogonal to one another. The Z axis positive direction side is the distal end side (distal side) to be inserted to a body, and the Z axis negative direction side is the proximal end side (proximal side, near side) to be manipulated by a technician such as a doctor. FIG. 2 is a side view illustrating the external appearance configuration of a torque device 100 of FIG. 1. FIG. 2 illustrates the external appearance configuration of the torque device 100, viewed from the X-axis positive direction. In the torque device 100 and the components thereof, a part including the distal end and extending from the distal end to a midway position toward the proximal end side is referred to as a "distal end portion". Similarly, a part including the proximal end and extending from the proximal end to a midway position toward the distal end side is referred to as a "proximal end portion".

The torque device 100 is a device for gripping a guide wire GW, and includes a main body 10 and a fixing tool 20. In a state where the guide wire GW is not fixed, the fixing tool 20 is at a given position (hereinafter, referred to as a "first position"). This state is the first state of the torque device 100. The torque device 100 is an example of the medical tool gripper in claims, and the guide wire GW is an example of the long medical tool in claims.

### A-2. Configurations of main body 10 and fixing tool 20:

The main body 10 is a substantially hollow cylindrical member with substantially linear main body inner space IS in which the guide wire GW can be arranged. The fixing tool 20 is a member for fixing the guide wire GW to the main body 10. The examples of the material forming the main body 10 and the fixing tool 20 include resin materials such as ABS resin and polyethylene. It is particularly preferable to use ABS resin that is light weighted, less deformed, and has high strength.

The main body inner space IS is extended over the entire length in the longitudinal direction (the Z axis direction) of the main body 10. The main body 10 has a main body groove G1 that is open on the outer periphery of the main body 10 and is communicated with the main body inner space IS over the entire longitudinal length. The width of the main body groove G1 (the width in the X axis direction) is larger than the diameter of the guide wire GW. Thus, the guide wire GW displaceable in the radial direction of the guide wire GW can be inserted to the main body groove G1.

The main body 10 has a fixing tool storing groove G2 that is communicated with the main body groove G1 and in which the fixing tool 20 can be stored. In the main body groove G1 and the fixing tool storing groove G2, the surfaces along the depth direction are the side surfaces of the grooves. The side surfaces of the main body groove G1 and the fixing tool storing groove G2 are both planar from the edge of the outer peripheral surface of the opening toward the main body inner space IS. The "opening" here indicates an opening of the groove of the main body 10 onto the outer peripheral surface.

As illustrated in FIG. 1 and FIG. 2, the main body 10 includes a distal end portion 11 including the distal end of the main body 10, a main portion 12 positioned on the proximal end side of the distal end portion 11, and a proximal end portion 13 positioned on the proximal end side of the main portion 12. The distal end portion 11 and the proximal end portion 13 are substantially hemispherical. The main portion 12 is hollow cylindrical with a substantially uniform diameter over the entire length in the longitudinal direction (the Z axis direction) of the main body 10.

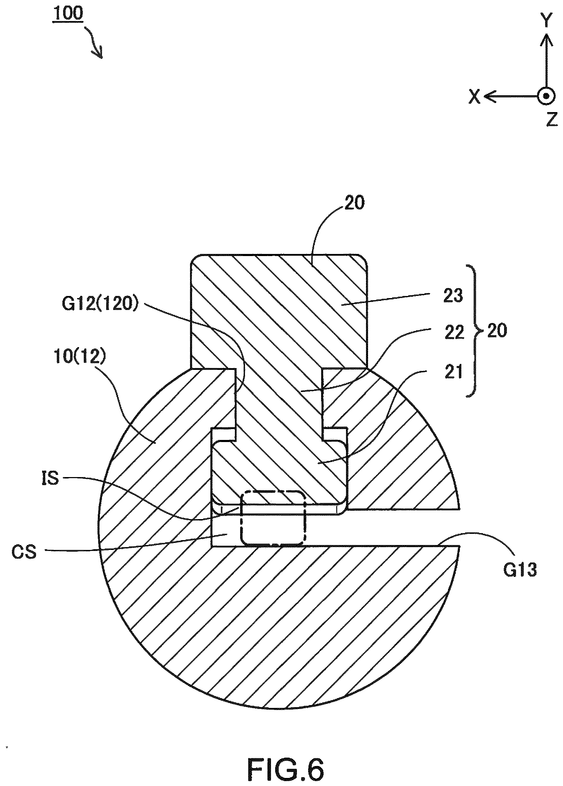

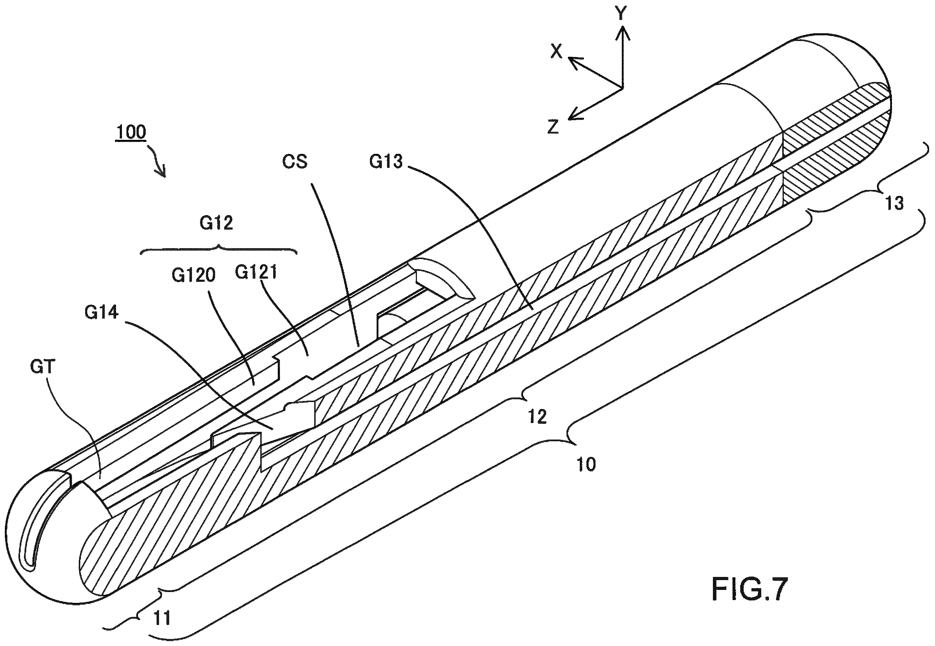

The main body 10 has the main body inner space IS, center space CS (see FIG. 6 and FIG. 7) communicated with the space forming the main body groove G1 and the fixing tool storing groove G2, a distal end groove G11, a rail groove G12, a proximal end groove G13, and a communication groove G14. The center space CS is formed in the main body 10 from the distal end of the distal end portion 11 to the proximal end of the proximal end portion 13. The center space CS is positioned in the substantially center of the main body, viewed from the longitudinal direction (viewed from the Z axis direction) of the main body 10. The width of the center space CS is larger than the diameter of the guide wire GW.

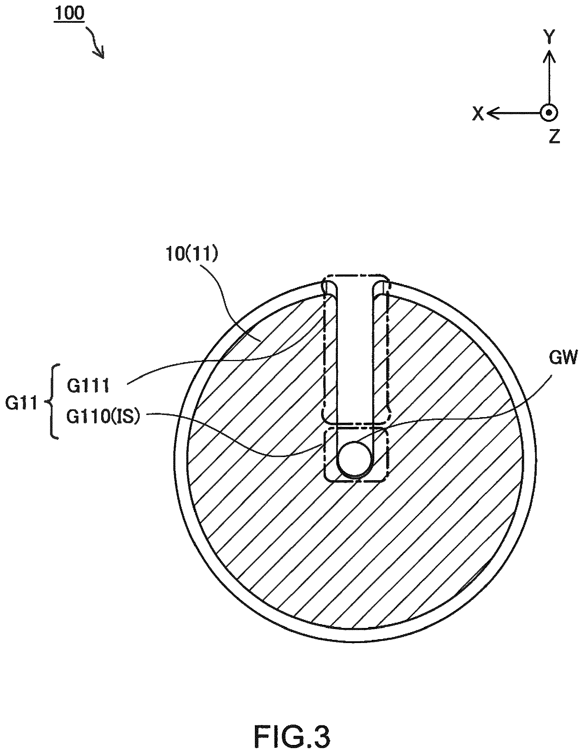

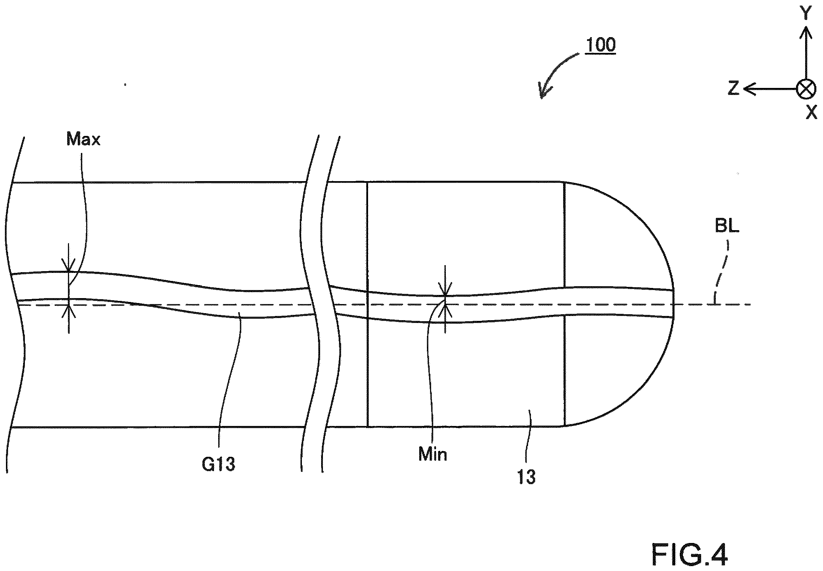

FIG. 3 is a transverse sectional view of the torque device 100 along III-III of FIG. 2. FIG. 4 is a diagram for explaining the form of the opening of the groove along the longitudinal direction of the main body 10. As illustrated in FIG. 2, the distal end groove G11 is formed from the distal end to the proximal end of the distal end portion 11 in the main body 10, and is a groove with a given direction substantially orthogonal to the longitudinal direction of the main body 10 (the Y axis negative direction) being a depth direction, as illustrated in FIG. 3. It is preferable that the opening of the distal end groove G11 is extended linearly in the longitudinal direction (the Z axis direction) of the main body 10. As illustrated in FIG. 4, it may be considered that "the opening of the groove A is extended linearly in the longitudinal direction of the main body 10" here indicates the satisfaction of the condition that with a reference line BL that is positioned in the center of the width at the proximal end position of the opening on the peripheral surface of the main body 10 and is along the longitudinal direction of the main body 10, a difference between a minimum value Min and a maximum value Max of the gap distance between a peripheral edge of the opening of the groove A and the reference line BL at an arbitral position in the longitudinal direction of the main body 10 is smaller than 100 µm (preferably smaller than 50 µm, and more preferably smaller than 30 µm). When a difference between the minimum value Min and the maximum value Max is 0, the reference line BL and the peripheral edge of the opening of the groove A along the longitudinal direction of the main body 10 is parallel to each other. FIG. 4 illustrates the degree of bending of (the peripheral edge of) the opening of the groove in an exaggerated manner to facilitate understanding, and illustrates examples of the minimum value Min and the maximum value Max in the opening of the proximal end groove G13. The groove is linear so that the guide wire GW is inserted linearly to the main body inner space IS. Therefore, the difference between the minimum value Min and the maximum value Max does not necessarily satisfy the above-described condition.

The width of the distal end groove G11 is slightly larger than the diameter of the guide wire GW. Thus, when the guide wire GW is arranged in the distal end groove G11, the position of the guide wire GW (more precisely, a part of the guide wire GW) is defined. As illustrated in FIG. 3, the bottom surface of the distal end groove G11 is positioned around the center of the main body 10, viewed from the longitudinal direction (viewed from the Z axis direction) of the main body 10. Thus, the guide wire GW is arranged on the bottom surface of the distal end groove G11 positioned in this manner, and thus positioned around the center of the main body 10, viewed from the longitudinal direction of the main body 10. In the distal end groove G11, a part that includes the bottom thereof and on which the guide wire GW is arranged is referred to as a "bottom portion G110 of the distal end groove G11", and the other part of the distal end groove G11 than the bottom portion G110 is referred to as a "non-bottom portion G111 of the distal end groove G11". The main body inner space IS is formed by space including the bottom portion G110 of the distal end groove G11 and the center space CS (more precisely, a part of the center space CS).

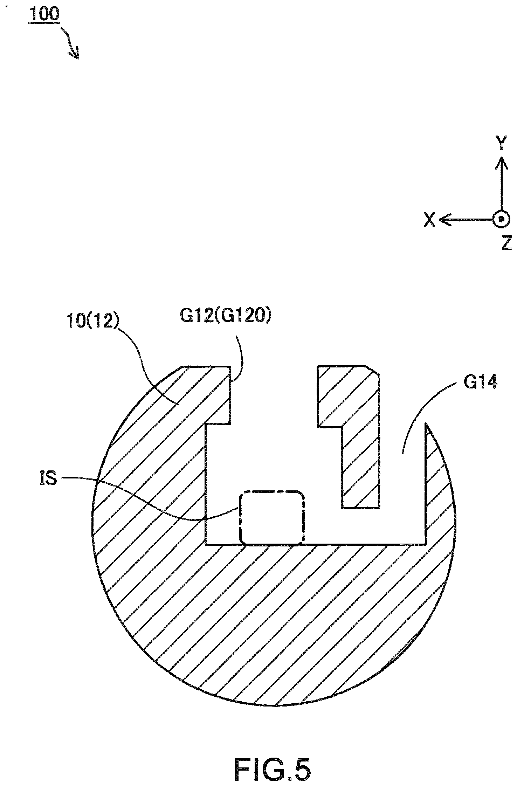

Next, the fixing tool 20 will be described. FIG. 5 is a transverse sectional view along IV-IV of FIG. 2. FIG. 6 is a transverse sectional view along V-V. The rail groove G12 is used to arrange the fixing tool 20 to be slidable in both directions along the longitudinal direction (the Z direction) of the main body 10. As illustrated in FIG. 1 and FIG. 2, the rail groove G12 is formed from the distal end of the main portion 12 toward the proximal end side in the main body 10. The rail groove G12 is communicated with the distal end groove G11 on the proximal end side of the distal end groove G11. The width of the rail groove G12 is larger than the width of the distal end groove G11. As illustrated in FIG. 4 and FIG. 5, the rail groove 12 is a groove with a direction substantially orthogonal to the longitudinal direction of the main body 10, the direction substantially same as the depth direction of the distal end groove G11 (the Y axis negative direction) being a depth direction. As illustrated in FIG. 3, FIG. 5, and FIG. 6, the position of the opening of the rail groove G12 overlaps the position of the opening of the distal end groove G11 in the circumferential direction of the main body 10. Here, "the position of the opening of a groove A overlaps the opening of a groove B in the circumferential direction of the main body 10" indicates that the position of at least a part of the opening of the groove A overlaps the opening of the groove B in the circumferential direction of the main body 10.

As illustrated in FIG. 6, the fixing tool 20 includes an inner wide portion 21, a narrow portion 22, and an outer wide portion 23 that are arranged along the Y axis positive direction of the main body 10. The width in the X axis direction of the inner wide portion 21 is larger than the width of the narrow portion G120 (see FIG. 7 described later) of the rail groove G12. The width in the X axis direction of the narrow portion 22 is smaller than the width of the narrow portion G120. The width in the X axis direction of the outer wide portion 23 is larger than the width of the narrow portion G120. The width in the X axis direction of the inner wide portion 21 is smaller than the width of the wide portion G121 of the rail groove G12.

As illustrated in FIG. 1, the outer wide portion 23 includes a plurality of projections 230 and recesses 231 (four projections 230 and three recesses 231 in FIG. 1) arranged along the longitudinal direction (the Z axis direction) of the main body 10, and the projections 230 and the recesses 231 appear alternately. In the embodiment, the height of the projection 230 positioned on the most distal end side is higher than the height of the other projections 230. On the rail groove G12, the fixing tool 20 can be displaced between a first position where the fixing tool 20 is positioned when the guide wire GW is not fixed thereto and a second position (where the fixing tool 20 is positioned when the guide wire GW is fixed thereto).

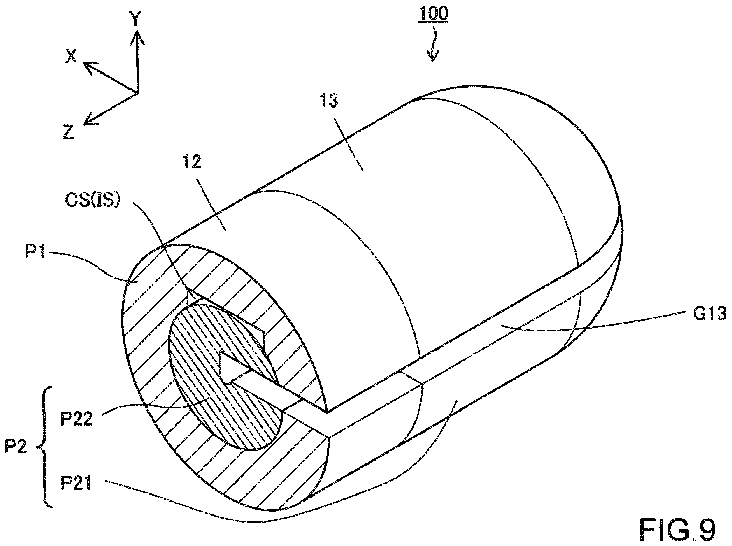

The fixing tool 20 is removable from the main body 10 under a certain condition, while it cannot be removed from the torque device 100 as a finished product. In a case where a second component P2 (see FIG. 9) described later is not inserted in the center space CS, that is, in a case where the second component P2 is removed from the main body 10, it is possible to remove the fixing tool 20 from the fixing tool storing groove G2 by sliding the fixing tool 20 along the rail groove G12 toward the proximal end side. However, in a case where the second component P2 is inserted in the center space CS, that is, in the case of the torque device 100 as a finished product, the second component P2 restricts sliding movement of the fixing tool 20. Thus, it is not possible to remove the fixing tool 20 from the fixing tool storing groove G2.

FIG. 7 is a perspective longitudinal sectional view of the torque device 100 illustrated in FIG. 1. For the purpose of explanation, the illustration of the fixing tool 20 is omitted. The rail groove G12 includes the narrow portion G120 communicated with the distal end groove G11 and the center space CS, and the wide portion G121 communicated with the narrow portion G120 on the proximal end side of the narrow portion G120. A groove formed by the distal end groove G11, the connection portion of the narrow portion G120 of the rail groove G2 with the communication groove G14, and the more distal end side than the connection portion (hereinafter, referred to as a "distal end portion GT of the main body groove G1") is an example of the distal end portion of the main body groove in claims.

The narrow portion G120 is communicated with the distal end groove G11 and the center space CS. As illustrated in FIG. 7, the narrow portion G120 extends linearly in the longitudinal direction (the Z axis direction) of the main body 10. As illustrated in FIG. 6, the width of the narrow portion G120 is larger than the width of the narrow portion 22 of the fixing tool 20 and smaller than the width of the inner wide portion 21 of the fixing tool 20. Thus, when the fixing tool 20 arranged in the narrow portion G120 of the rail groove G12 is about to be displaced in a direction separating from the main body 10, the inner wide portion 21 of the fixing tool 20 comes into contact with the wall portion of the main body 10, thereby preventing the fixing tool 20 from being removed from the main body 10. In this manner, the fixing tool 20 is held in the main body 10. The width of the narrow portion G120 is larger than the width of the distal end groove G11. Thus, the guide wire GW can be inserted to the narrow portion G120.

The wide portion G121 is communicated with the narrow portion G120 and the center space CS. The wide portion G121 is extended linearly in the longitudinal direction (the Z axis direction) of the main body 10. As illustrated in FIG. 6, the width of the wide portion G121 is larger than the narrow portion G120 and larger than the width of the inner wide portion 21 of the fixing tool 20. In this manner, as is understood from FIG. 1 and FIG. 7, a part of the fixing tool 20 (the inner wide portion 21 and a part of the narrow portion 22) is arranged in the wide portion G121 with a large width, and the fixing tool 20 is slid along the narrow portion G120 toward the distal end side, whereby it is possible to arrange the fixing tool 20 at the distal end of the narrow portion G120.

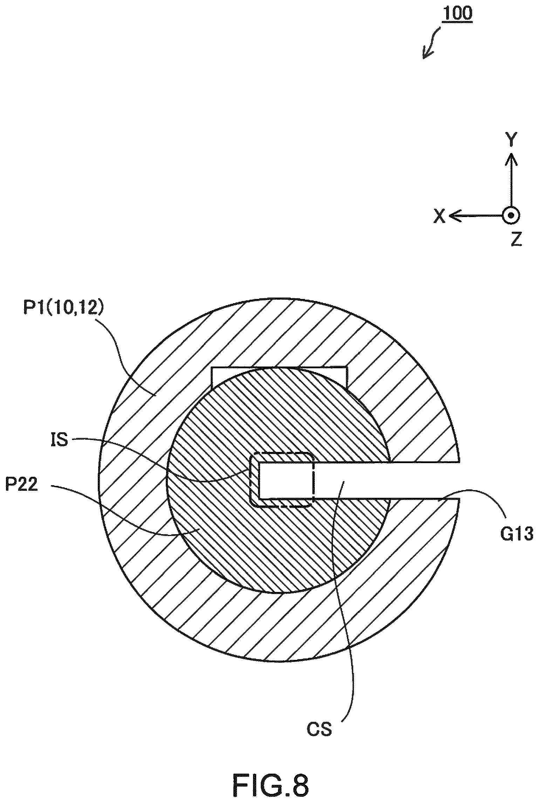

As illustrated in FIG. 2, the proximal end groove G13 is formed from the proximal end of the main portion 12 toward the distal end side in the main body 10. FIG. 8 is a transverse sectional view along VII-VII of FIG. 2. As is understood from FIG. 3 and FIG. 8, the openings of the proximal end groove G13 and the distal end groove G11 are at different positions in the circumferential direction of the main body 10. That is, viewed from the longitudinal direction (the Z axis direction) of FIG. 3 and FIG. 8, the positions of the openings of the proximal end groove G13 and the distal end groove G11 do not overlap each other. The proximal end groove G13 is a groove with a direction substantially orthogonal to the longitudinal direction of the main body 10, the direction orthogonal to the depth direction of the distal end groove G11 and the longitudinal direction of the main body 10 (the X axis positive direction) being a depth direction. The proximal end groove 13 is an example of the proximal end portion of the main body groove in claims.

As illustrated in FIG. 1, FIG. 2, and FIG. 7, the communication groove G14 is formed in the main portion 12 of the main body 10. The communication groove G14 is communicated with the narrow portion G120 of the rail groove G12 and the proximal end groove G13. The communication groove G14 is a groove with a direction substantially orthogonal to the longitudinal direction of the main body 10, the direction substantially same as the depth direction of the distal end groove G11 (the Y axis negative direction) being a depth direction. The communication groove G14 is an example of the communication portion in claims.

The communication groove G14 is connected with the proximal end side of the narrow portion G120 of the rail groove G12. The above-described fixing tool storing groove G2 is formed by the connection portion with the communication groove G14 and the more proximal end side than the connection portion in the narrow portion G120 of the rail groove G12, and the wide portion G121.

As illustrated in FIG. 1 and FIG. 7, the opening of the communication groove G14 is extended in a direction inclined relative to the longitudinal direction of the main body 10 so as to the closer to the proximal end of the main body 10, the closer a position of the opening of the communication groove G14 is to the opening of the proximal end groove G13. The width of the opening of the communication groove G14 is larger toward the proximal end groove G13. The main body groove G1 includes the non-bottom portion G111 of the distal end groove G11, the connection portion with the communication groove G14 and the more distal end side than the connection portion in the narrow portion G120 of the rail groove G12, the communication groove G14, and the proximal end groove G13.

FIG. 9 is a perspective transverse sectional view of the torque device 100, and is a transverse sectional view at a position on the proximal end side of the main part 12. As illustrated in FIG. 1, FIG. 2, and FIG. 9, the main body 10 includes a first component P1, and a second component P2 connected to the first component P1. The first component P1 and the second component P2 are mutually separate bodies. The first component P1 includes the distal end portion 11 and a part (positioned on the distal end side) of the main portion 12 of the main body 10. The second component P2 includes a part of the main portion 12 (a part positioned on the proximal end side) and the proximal end portion 13 of the main body 10. The first component P1 includes parts of the main body 10 where the center space CS, the distal end groove G11, the rail groove G12, the communication groove G14, and a part of the proximal end groove G13 (a part positioned on the distal end side) are formed. The second component P2 includes a part of the main body 10 where a part of the proximal end groove G13 (a part positioned on the proximal end side) is formed. As illustrated in FIG. 9, the second component P2 includes a base portion P21 with the substantially same diameter as the first component P1, and a small diameter portion P22 that is connected to the distal end side of the base portion P21 and has a smaller diameter than the base portion P21. The small diameter portion P22 is inserted in the main body inner space IS of the first component P1. The second component P2 can be fixedly attached to the first component P1 by snap-fit or the like.

The second component P2 may be attached to the first component P1 to be rotatable in the circumferential direction of the main body 10. For example, a groove extending along the circumferential direction is formed on one of the inner peripheral surface of the first component P1 and the peripheral surface of the small diameter portion P22, and a projection loosely fitted to the groove is formed on the other surface, so that the projection is fitted to the groove. In this manner, the second component P2 is rotatable in the circumferential direction relative to the first component P1. The second component P2 is rotated such that the position of the proximal end groove G13 of the second component P2 is different in the circumferential direction from that of the proximal end groove G13 of the main portion 12, whereby the guide wire GW stored in the main portion 12 can be stored more easily in the proximal end groove G13 of the second component P2.

### A-3. Operation of first embodiment:

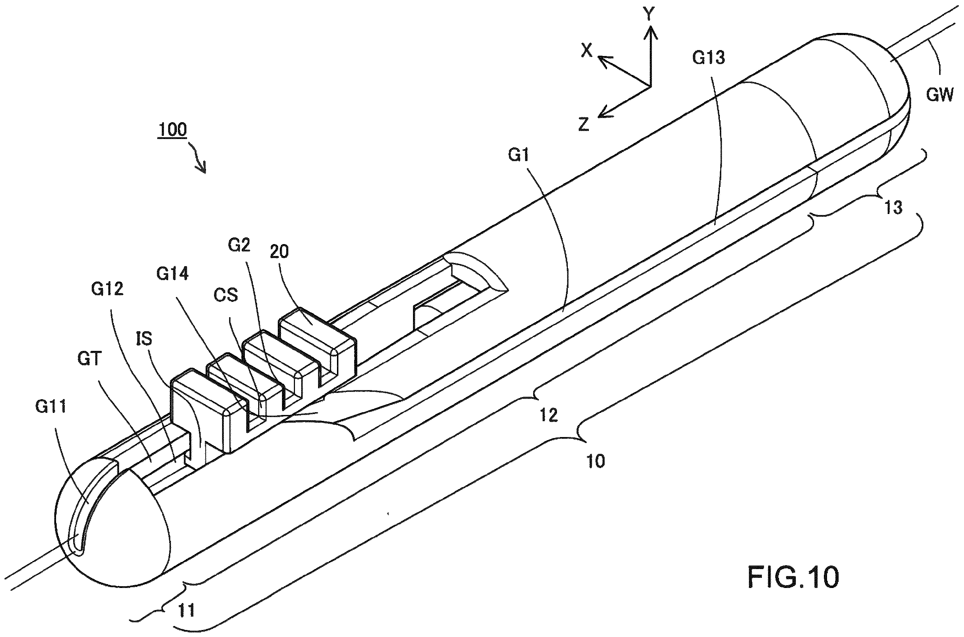

As described above, FIG. 1 illustrates the external appearance configuration of the torque device 100 in the first state according to the first embodiment, and FIG. 10 illustrates the external appearance configuration of the torque device 100 with the guide wire fixed thereto (hereinafter, referred to as a "second state") according to the first embodiment.

To attach (fix) the torque device 100 to the guide wire GW, the fixing tool 20 is first arranged at the first position illustrated in FIG. 1 to be stored in the wide portion G121 of the fixing tool storing groove G2. Next, (a part of) the guide wire GW is arranged in the main body inner space IS through the main body groove G1. Here, the guide wire GW is inserted into the main body groove G1 while displacing the guide wire GW in the radial direction.

If the torque device has the main body inner space IS for storing the guide wire GW but does not have an opening communicated with the main body inner space IS, in order to fix the guide wire GW to the main body 10, the distal end of the guide wire GW is inserted in the main body inner space IS from the proximal end (or the distal end) of the main body 10, and the guide wire GW or the main body 10 itself is moved in the longitudinal direction thereof, so that a part of the guide wire GW to be arranged in the main body 10 is arranged in the main body inner space IS. Here, in the operation of arranging the guide wire GW in the main body inner space IS, it requires work to move the guide wire GW in the longitudinal direction because the guide wire GW is long. However, in the torque device 100 of the first embodiment, a part of the guide wire GW to be arranged in the main body 10 is inserted into the main body groove G1 by inserting the part into the opening of the main body groove G1 from the side surface of the main body 10, so as to be arranged in the main body inner space IS. Thus, it is possible to improve the efficiency of the operation of arranging the guide wire GW in the main body inner space IS.

Next, the fixing tool 20 is moved along the longitudinal direction (the Z axis positive direction) of the rail groove G12, and arranged at the second position in the main body groove G1. Here, a part of the fixing tool 20 is positioned in the main body groove G1, and the inner wide portion 21 of the fixing tool 20 and the main body 10 sandwich the guide wire GW to fix the guide wire GW to the main body 10 (see FIG. 6).

As described above, among a plurality of projections 230 formed on the fixing tool 20, the height of the projection 230 positioned on the most distal end side is higher than the height of the other projections 230. Thus, it is possible to easily move the fixing tool 20 in the longitudinal direction (the Z axis direction) of the main body 10 by contact of a technician's finger with the projection 230 positioned on the most distal end side.

To release fixing of the guide wire GW by the fixing tool 20, the fixing tool 20 is restored in the first position to be stored in the fixing tool storing groove G2. In this manner, the state of the guide wire GW sandwiched by the inner wide portion 21 of the fixing tool 20 and the main body is released, and thus the fixing of the guide wire GW is released.

### A-4. Effect of first embodiment

As described above, in the torque device 100 of the first embodiment, the substantially hollow cylindrical main body 10 has the main body inner space IS that extends over the entire longitudinal length of the main body 10 and in which the guide wire GW can be arranged, and the main body groove G1 that extends over the entire length in the longitudinal direction (the Z axis direction) of the main body 10, is communicated with the main body inner space IS, and is open on the outer periphery of the main body 10 is formed. In the main body groove G1, the positions of the openings of the distal end groove G11 and the proximal end groove G13 are different in the circumferential direction of the main body 10.

In the torque device in which the opening of the main body groove G1 is formed linearly over the entire longitudinal length of the main body 10 on the outer periphery of the main body 10, in a case where the guide wire GW is fixed to the main body 10 by the fixing tool 20, the rear end portion of the guide wire GW may come out to the outside of the main body 10 through the main body groove G1 against the intention of a technician, which may impede the operation of the guide wire GW or the like by the technician. Meanwhile, in the torque device 100, the positions of the openings of the distal end groove G11 and the proximal end groove G13 are different in the circumferential direction of the main body 10, whereby it is possible to suppress the problem that the rear end portion of the guide wire GW comes out to the outside of the torque device 100 through the main body groove G1, as compared with the case in which the opening of the main body groove G1 is formed linearly over the entire longitudinal length of the main body 10 on the outer periphery of the main body 10. Thus, the torque device 100 suppresses the case in which a part of the guide wire GW comes out to the outside of the main body 10 through the main body groove G1 against the intension of a technician. Therefore, it is possible to prevent the operation of the guide wire GW against the intention of a technician.

In the torque device 100, the main body 10 has the fixing tool storing groove G2 that is communicated with the main body groove G1 and stores the fixing tool 20 such that the fixing tool 20 is not positioned in the main body groove G1. When the guide wire GW is arranged in the main body inner space IS through the main body groove G1, the fixing tool 20 is stored in the fixing tool storing groove G2. Thus, the operation of arranging the guide wire GW is not impeded.

In the torque device 100, the openings of the distal end portion GT and the proximal end groove G13 of the main body groove G1 are linear along the longitudinal direction (the Z axis direction) of the main body 10. The main body groove G1 includes the communication groove G14 communicating the openings of the distal end portion GT and the proximal end groove G13. In this manner, the guide wire GW is arranged in the main body inner space IS through the main body groove G1 having such a configuration, whereby it is possible to more reliably secure the straightness of the guide wire GW arranged in the main body inner space IS.

In the torque device 100, the opening of the communication groove G14 is extended in a direction inclined relative to the longitudinal direction of the main body 10 such that the closer to the proximal end groove G13, the closer the position of the opening in the direction orthogonal to the longitudinal direction of the main body 10 (the X axis direction in the figure) is to the opening of the proximal end portion of the main body groove G1. If the opening of the communication groove G14 is extended in a direction orthogonal to the longitudinal direction of the main body 10, viewed from the depth direction of the distal end portion GT of the main body groove G1, the guide wire GW is bent at a sharp angle (substantially a right angle) when the guide wire GW is inserted into the main body groove G1, which may deteriorate the straightness of the guide wire GW. Meanwhile, in the torque device 100, the opening of the communication groove G14 is extended in the inclined direction as described above. Thus, when the guide wire GW is inserted into the main body groove G1, the bent angle of the guide wire GW is relatively small. Therefore, in the torque device 100, when the guide wire GW is inserted into the main body groove G1, it is possible to suppress the deterioration of the straightness of the guide wire GW and more reliably secure the straightness of the guide wire GW arranged in the main body inner space IS.

In the torque device 100, the main body 10 includes the first component P1 that includes the distal end of the main body 10 and where the distal end portion GT of the main body groove G1 is formed, and the second component P2 connected to the first component P1 that includes the proximal end of the main body 10 and where the proximal end groove G13 is formed. If the main body 10 is produced as an integrated object, easy production is not possible because the main body inner space IS and the main body groove G1 are difficult to form, for example. However, in the torque device 100, the main body 10 is formed by the separate components of the first component P1 and the second component P2. Therefore, it is possible to produce the torque device 100 by forming the distal end portion GT of the main body groove G1 in the first component P1 and the proximal end groove G13 of the main body groove G1 in the second component P2, and then connecting the first component P1 and the second component P2. In this manner, it is possible to consider that the torque device 100 may facilitate the production, as compared with the configuration in which the main body 10 is produced as an integrated object.

### B. Second embodiment

### B-1. Configuration of torque device 100A

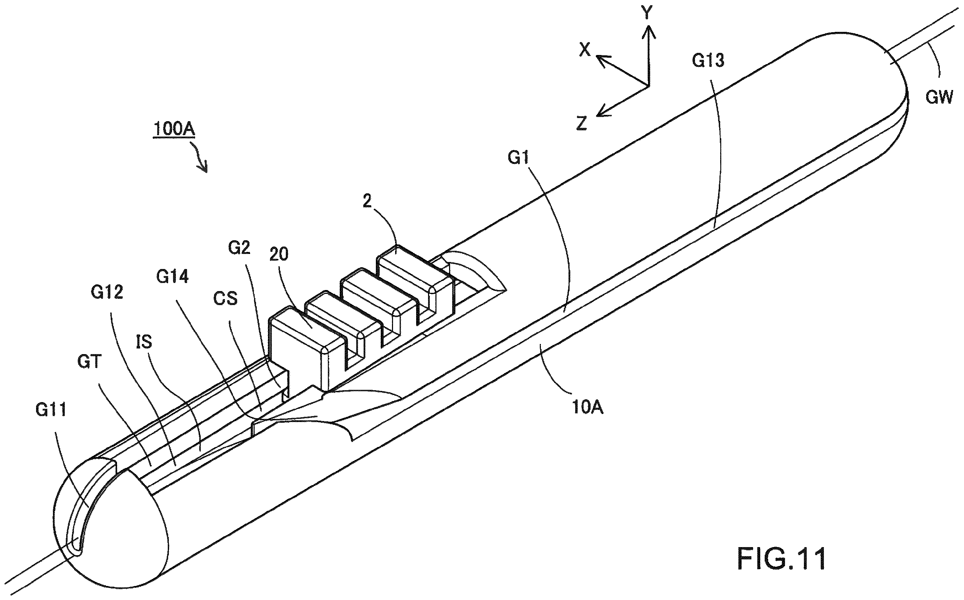

FIG. 11 is a perspective view illustrating an external appearance configuration of a torque device according to the second embodiment. A torque device 100A of the second embodiment is different from the torque device 100 of the first embodiment in the aspect that a main body 10A is integrally molded without a plurality of separate members that are separate from each other. Hereinafter, any component that is the same as that of the torque device 100 of the above-described first embodiment, in the components of the torque device 100A of the second embodiment, the explanation thereof is omitted as appropriate by marking it with the same symbol.

As described above, the main body 10A of the second embodiment is a member formed integrally by molding. Except such an aspect, the main body 10A has the same shape as the main body 10. That is, the main body 10A has inner space and grooves such as the main body inner space IS and the main body groove G1 formed in the main body 10 of the first embodiment. Moreover, the effects of the inner space and grooves are same as those in the first embodiment, and the detailed explanation thereof is omitted.

In the torque device 100A, the fixing tool 20 is arranged in the fixing tool storing groove G2 by pushing the inner wide portion 21 of the fixing tool 20 into the rail groove G12 of the main body 10A and fitting it in the rail groove G12. Although an operator such as a technician cannot easily remove the fixing tool 20 from the main body 10A, the probability of losing the fixing tool 20 as a part is eliminated.

### B-2. Effect of second embodiment:

In the torque device 100A, the main body 10A is formed integrally by molding. Thus, it is possible to reduce the number of members as compared with the case in which the main body 10A is formed by a plurality of separate members. Other effects of the second embodiment are similar to the effects of the first embodiment, and thus the detailed explanation thereof is omitted.

### C. Third embodiment

### C-1. Configuration of torque device 100B

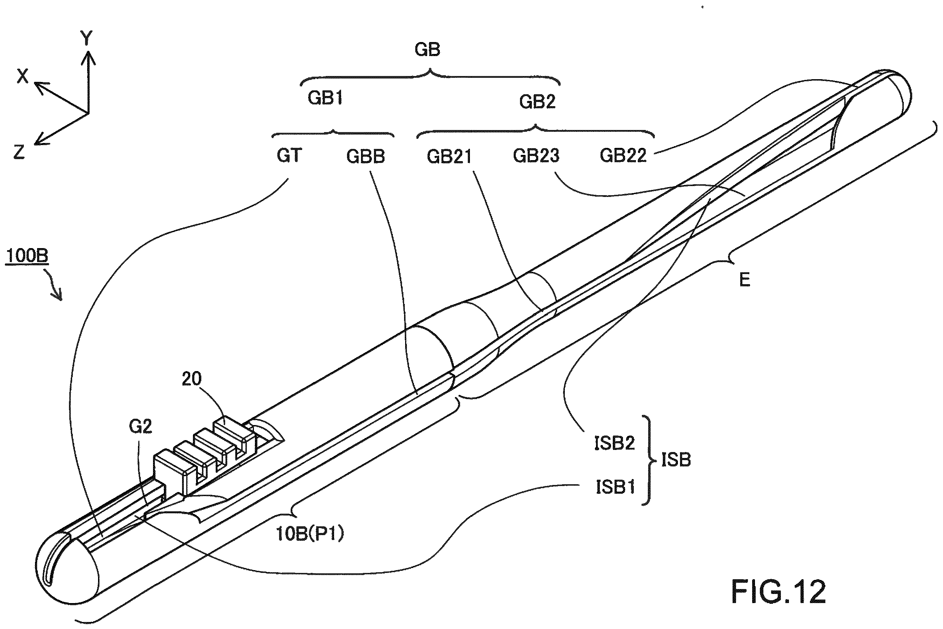

FIG. 12 is a perspective view illustrating an external appearance configuration of a torque device 100B according to the third embodiment. The torque device 100B is different from the torque device 100 of the first embodiment in the aspect that an extension member E is provided instead of the second component P2. Hereinafter, any component that is the same as that of the torque device 100 of the above-described first embodiment, in the components of the torque device 100B of the third embodiment, the explanation thereof is omitted as appropriate by marking it with the same symbol.

The torque device 100B of the third embodiment includes a main body 10B formed by the first component P1 of the first embodiment and the extension member E connected to the proximal end side of the main body 10B. The extension member E is attached to the main body 10B to be rotatable in the Z axis rotating direction and removable.

The main body 10B has main body inner space ISB1 having the substantially same configuration as the main body inner space IS of the first embodiment, and a main body groove GB1 having the substantially same configuration as the main body groove G1 of the first embodiment.

The main body inner space ISB1 is different from the main body inner space IS of the first embodiment only in the aspect that the length of the proximal end portion (the longitudinal length of the main body 10B) is shorter by the second component P2 not provided. That is, it is considered that the torque device formed by removing the second component P2 from the torque device 100 of the first embodiment and attaching the extension member E is the torque device 100B of the third embodiment. The other components in the third embodiment are similar to those in the first embodiment.

The main body groove GB1 includes the distal end portion GT including the distal end of the main body groove GB1, and a proximal end portion GBB including the proximal end of the main body groove GB1. The position of the opening of the proximal end portion GBB is different in the circumferential direction of the main body 10B (the circumferential direction around the Z axis) from the position of the opening of the distal end portion GT. That is, viewed from the circumferential direction of the main body 10B, the positions of the openings of the proximal end portion GBB and the distal end portion GT do not overlap each other. The main body groove GB1 is different from the main body groove G1 of the first embodiment only in the aspect that the length of the proximal end portion GBB (the longitudinal length of the main body 10B) is shorter than the length of the proximal end groove G13 of the first embodiment by the second component P2 not provided.

The extension member E is a substantially hollow cylindrical member connected to the proximal end side of the main body 10B. The extension member E has extension inner space ISB2 that extends over the entire length in the longitudinal direction (the Z axis direction) of the main body 10B and is substantially linear inner space in which the guide wire GW can be arranged, and an extension groove GB2 that can be communicated with the opening of the main body 10B and is communicated with the extension inner space ISB2 over the entire length in the longitudinal direction of the main body 10B.

The extension groove GB2 includes a distal end portion GB21 including the distal end of the extension groove GB2, a proximal end portion GB22 including the proximal end of the extension groove GB2, and a communication portion GB23 communicating the distal end portion GB21 and the proximal end portion GB22.

The distal end portion GB21 of the extension groove GB2 is communicated with the proximal end portion GBB of the main body groove GB1.

The openings of the distal end portion GB21 and the proximal end portion GB22 of the extension groove GB2 are both linear along the longitudinal direction (the Z axis direction) of the main body 10B, and the depth direction of the groove is linear from the opening on the outer peripheral surface toward the extension inner space ISB2.

The position of the opening of the proximal end portion GB22 of the extension groove GB2 is different in the circumferential direction of the main body 10B (the circumferential direction around the Z axis) from the position of the opening of the proximal end portion GBB of the main body groove GB1. That is, viewed from the circumferential direction of the main body 10B, the positions of the openings of the proximal end portion GB22 of the extension groove GB2 and the proximal end portion GBB of the main body groove GB1 do not overlap each other. In FIG. 12, the position of the opening of the proximal end portion GB22 of the extension groove GB2 overlaps the position of the opening of the distal end portion GT of the main body groove GB1 in the circumferential direction of the main body 10B. As the material forming the extension member E, the same material as the material forming the main body 10 or the fixing tool 20, for example, can be used.

The operation of the third embodiment are same as those of the first embodiment, and the detailed explanation thereof is omitted. (A part of) the guide wire GW is arranged in the inner space ISB formed by the main body inner space ISB1 and the extension inner space ISB2 through the groove GB formed by the main body groove GB1 and the extension groove GB2.

### C-2. Effect of third embodiment

With the same reason for the torque device 100 of the first embodiment, the torque device 100B of the third embodiment also suppresses the case in which a part of the guide wire GW comes out to the outside of the main body 10 through the main body groove GB1 against the intention of a technician, thus impeding the operation of the guide wire GW or the like by the technician.

Furthermore, the torque device 100B includes the substantially hollow cylindrical extension member E connected to the proximal end side of the main body 10B. The extension member E has the extension inner space ISB2 that extends over the entire length in the longitudinal direction (the Z axis direction) of the main body 10B and in which the guide wire GW can be arranged, and the extension groove GB that is open on the outer periphery of the main body 10B and is communicated with the extension inner space ISB2 over the entire length in the longitudinal direction of the main body 10B. The position of the opening of the proximal end portion GB22 of the extension groove GB2 is different in the circumferential direction of the main body 10B (the circumferential direction around the Z axis) from the position of the opening of the proximal end portion GBB of the main body groove GB1. With such an extension member E, it is possible to increase the length of the torque device 100B itself. Furthermore, the position of the opening of the proximal end portion of the torque device 100B with the extension member E attached thereto (the proximal end portion GB22 of the extension groove) is different from the position of the opening of the proximal end portion of the main body 10B without the extension member E attached thereto. Thus, it is possible to more securely suppress the problem that the rear end portion of a medical tool comes out to the outside of the torque device 100B through the main body groove GB1. If the position of the opening of the proximal end portion GB22 of the extension groove GB2 overlaps the position of the opening of the distal end portion GT of the main body groove GB1 in the circumferential direction of the main body 10B, it is possible to more reliably secure the straightness of the guide GW when arranged in the above-described inner space ISB through the groove GB formed by the main body groove GB1 and the extension groove GB2.

### D. Modification example:

The technology disclosed herein is not limited to the above-described embodiments, and can be modified in various forms without departing from the gist thereof. For example, the following modification examples are also possible.

The configurations of the torque devices 100, 100A, and 100B in the above embodiments are only examples and can be modified in various forms.

In the above embodiments, the extending direction of the openings of the grooves (the main body grooves G1, GB1, the fixing tool storing groove G2, and the like) is not particularly limited. The openings of the grooves may not be linear (may be curved, for example).

In the above embodiments (or modification examples, the same applies hereinafter), the depth direction of the grooves is not particularly limited. In each groove, the side surface of the groove along the depth direction may not be planner, and may be curved in the depth direction.

In the torque devices 100, 100B of the above embodiments, the fixing tool 20 is removable from the first position under a certain condition. However, the fixing tool 20 may constantly remain in the fixing tool storing groove G2.

In the above-described third embodiment, the main body 10B may be formed by a plurality of parts that are separate from each other.

In the torque device 100B of the above-described third embodiment, the extension member E connected to the proximal end side of the main body 10B may be rotatable in the circumferential direction of the extension member E, and the angles of the grooves and inner space formed in the extension member E may be changed. Moreover, in the form illustrated in FIG. 12, the outer diameter of the extension member E is partially smaller than that of the main body 10B. However, the outer diameter of the extension member E may be the same as that of the main body 10B over the entire longitudinal direction, or may be partially larger along the longitudinal direction.

The materials for members forming the torque devices 100, 100A, and 100B in the above embodiments are only examples, and may be modified variously.

The above embodiments have described, as an example, the torque device for gripping a guide wire. However, the disclosed embodiments are not limited to the torque device, and can be applied in the same manner to any medical tool gripper for gripping a long medical tool.

### REFERENCE SIGNS LIST

- 10: main body (of first embodiment)
- 10A: main body (of second embodiment)
- 10B: main body (of third embodiment)
- 20: fixing tool
- 100: torque device (of first embodiment)
- 100A: torque device (of second embodiment)
- 100B: torque device (of third embodiment)
- GW: guide wire
- P1: first component
- P2: second component

## Claims

1. A medical tool gripper for gripping a long medical tool, the medical tool gripper comprising:
a substantially hollow cylindrical main body that includes main body inner space in which the medical tool can arranged; and
a fixing tool that fixes the medical tool to the main body, wherein
the main body has a main body groove that extends over an entire longitudinal direction of the main body, is communicated with the main body inner space, and is open on an outer periphery of the main body, and
the main body groove includes
a distal end portion including a distal end of the main body groove and
a proximal end portion including a proximal end of the main body groove and having an opening at a different position from an opening of the distal end portion in a circumferential direction of the main body.

2. The medical tool gripper according to claim 1, wherein
the opening of the distal end portion and the opening of the proximal end portion of the main body groove are linear along the longitudinal direction, and
the main body groove further includes a communication portion communicating the opening of the distal end portion and the opening of the proximal end portion.

3. The medical tool gripper according to claim 2, wherein
an opening of the communication portion is extended in a direction inclined relative to the longitudinal direction such that the closer to the proximal end of the main body, the closer a position of the opening in a direction orthogonal to the longitudinal direction is to the opening of the proximal end portion of the main body groove.

4. The medical tool gripper according to any one of claims 1 to 3, wherein
the main body includes
a first component that includes a distal end of the main body and in which the distal end portion of the main body groove is formed, and
a second component connected to the first component that includes a proximal end of the main body and in which the proximal end portion of the main body groove is formed.

5. The medical tool gripper according to any one of claims 1 to 3, wherein
the main body is formed integrally by molding.

6. The medical tool gripper according to any one of claims 1 to 5_{.} further comprising:
a substantially hollow cylindrical extension member that is connected to a proximal end side of the main body, wherein
the extension member includes
extension inner space that extends over the entire longitudinal length and in which the medical tool can be arranged and
an extension groove that is open on the outer periphery of the main body, and is communicated with the extension inner space over the entire longitudinal length of the main body, and
a position of an opening of a proximal end portion of the extension groove is different in the circumferential direction of the main body from a position of an opening of the proximal portion of the main body groove.
